(19) Europäisches Patentamt
European Patent Office
Office européen des brevets

(11) **EP 2 034 901 B1**

(12) **EUROPEAN PATENT SPECIFICATION**

(45) Date of publication and mention
of the grant of the patent:
**22.03.2017 Bulletin 2017/12**

(51) Int Cl.:
***A61B 8/08*** *(2006.01)* ***A61B 5/02*** *(2006.01)*

(21) Application number: **07748596.9**

(22) Date of filing: **19.06.2007**

(86) International application number:
**PCT/SE2007/050436**

(87) International publication number:
**WO 2008/002257 (03.01.2008 Gazette 2008/01)**

(54) **GLOBAL AND LOCAL DETECTION OF BLOOD VESSEL ELASTICITY**

GLOBALER UND LOKALER NACHWEIS VON BLUTGEFÄSS-ELASTIZITÄT

DÉTECTION GLOBALE ET LOCALE DE L'ÉLASTICITÉ D'UN VAISSEAU SANGUIN

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.06.2006 SE 0601432**

(43) Date of publication of application:
**18.03.2009 Bulletin 2009/12**

(73) Proprietor: **Brodin, Lars-Åke**
**187 35 Täby (SE)**

(72) Inventors:
• **ELMQVIST, Håkan**
**S-167 62 Bromma (SE)**
• **BJÄLLMARK, Anna**
**S-169 58 Solna (SE)**
• **LARSSON, Matilda**
**S-126 37 Hägersten (SE)**
• **BRODIN, Lars-Ake**
**S-187 35 Täby (SE)**

(74) Representative: **Brann AB**
**P.O. Box 3690**
**Drottninggatan 27**
**103 59 Stockholm (SE)**

(56) References cited:
EP-A2- 1 475 040     WO-A1-2005/074804
US-A1- 2001 016 686   US-A1- 2005 124 892
US-B1- 6 654 628

• **NIKI K ET AL: "A new noninvasive measurement system for wave intensity: evaluation of carotid arterial wave intensity and reproducibility", HEART AND VESSELS, SPRINGER, BERLIN, DE, vol. 17, 1 January 2002 (2002-01-01), pages 12-21, XP003018367, ISSN: 0910-8327, DOI: 10.1007/S003800200037**
• **NIKI K. ET AL.: 'A new noninvasive measurement system for wave intensity: evaluation of carotid arterial wave intensity and reproducibility' HEART VESSELS vol. 17, 2002, pages 12 - 21, XP003018367**
• **CINTHIO M. ET AL.: 'Longitudinal movements and resulting shear strain of the aerterial wall' AM. J. PHYSIOL. HEART CIRC. PHYSIOL. vol. 291, 2006, pages H394 - H402, XP003018368**
• **SUNAGAWA K. ET AL.: 'Simultaneous measurement of blood flow and arterial wall vibrations in radial and axial directions' ULTRASONICS SYMPSIUM, IEEE vol. 2, 2000, pages 1541 - 1544, XP010540908**
• **SUNAGAWA K ET AL: "Simultaneous measurement of blood flow and arterial wall vibrations in radial and axial directions", PROCEEDINGS / 2000 IEEE ULTRASONICS SYMPOSIUM : OCTOBER 22 - 25, 2000, HOTEL CARIBE HILTON, SAN JUAN, PUERTO RICO ; AN INTERNATIONAL SYMPOSIUM, IEEE SERVICE CENTER, PISCATAWAY, NJ, vol. 2, 22 October 2000 (2000-10-22), pages 1541-1544, XP010540908, ISBN: 978-0-7803-6365-6**

**Description**

The present invention relates to a system according to the preamble of claim 1.

**General description**

Atherosclerosis is characterized of more or less local growth of the inner layer of the artery wall. The blood has increased difficulties of passing due to the constriction caused by the fat layers on the inside of the vessels. This phenomenon primarily occurs in the large and middle-sized arteries, e.g. aorta, the coronary heart vessels, or the large vessels to the bones or neck.

Ischemic situations and heart infarcts are generally caused by disturbances of the blood flow due to atherosclerotic constrictions. Since atherosclerosis makes the vessel wall thicker and it looses its flexibility, reduced arterial elasticity is an indicator of atherosclerosis [1,2]. Arterial elasticity may be measured in different ways, where some is based upon the by Moens-Korteweg's equation for Pulse Wave Velocity (PWV). Moens-Korteweg's equation describes the following relationship:

$$PWV = \sqrt{Eh/2r\rho}$$

where PWV is the velocity of the pulse wave, E is the module of elasticity, h is the wall thickness, r is the vessel radius, and $\rho$ is the density of blood. The pulse wave velocity increases by the rigidity of the blood vessel and the pulse wave velocity for a healthy person is rarely above 10 m/s. Most of the presently used methods compares the time delay of the pulse wave between two measurement points. If the measurement points are widely separated, e.g. in aorta and in A. radialis, the pulse wave velocity describes the global elasticity of the vessel. To obtain an indication of the local elasticity of the blood vessel, e.g. close to an atherosclerotic constriction, the measurement points must be located much closer each other. Rabben et al [3] introduced a new method to locally measure arterial elasticity. The problems of above methods are that they are based upon discrete dynamic events, e.g. the contraction of the heart. In order to also take into account cooperating parts of the circulatory system, i.e. include the relationship between the heart's contraction ability and the blood vessel elasticity in the analysis, Parker et al introduced in 1990 a completely new concept - Wave Intensity Analysis [4].

Wave Intensity Analysis is a general name of analysis of wave movements in elastic blood vessels. For many years the research of arterial waves has been concentrated on various impedance techniques [5,6] here the basic theory has been that arterial waves may be expressed by a sum of sinus wave having different amplitude and frequency. In Wave Intensity Analysis this assumption is disregarded and instead a wave is described as the sum of sequential infinitesimal wave fronts, where one wave front is represented by a temporal pressure difference (dP) or the flow velocity difference (dU) during a sample interval. This requires neither linearity nor periodicity and the result is presented in relation to time. dP is defined as P(t2)-P(t1) in N/m2 and dU is defined as U(t2)-U(tl) in m/s. In Wave Intensity Analysis the product of the pressure derivate and the flow velocity derivate is calculated and may be regarded as a measure of the wave's transported energy (intensity) per time and area unit (W/m2) according to the following relationship:

$$dI = dP*dU$$

By adjusting the intensity change (dI) with regard to a sample interval comparisons between different studies is simplified. The basis for making this adjustment is found in Niki et al [7] and the new value is expressed in the following way:

$$dI = dP/dt*dU/dt$$

However, the adjusted expression has no real physical meaning as its unit is W/m2s2 [8].

An arterial wave may be separated into forward propagating and backward propagating waves and the sign of the intensity change (dI) describes the wave direction. There are different techniques of separating forward and backward propagating waves. Usually, the wave is separated from a curve of the pressure as a function of the flow velocity. Parker's research team has worked in this field [4,8-10] during a long time and states that almost all important information may be obtained from the net intensity change since the time intervals of overlapping forward and backward propagating waves are relatively short. In practise this means that a separation of forward and backward propagating waves not is necessary in order to make an adequate judgement of blood vessel elasticity [9].

The graph of the wave intensity change (dI) has three characteristic peaks [9]. Initially is a positive peak representing a forward propagating wave where both pressure and flow velocity in the blood vessel increase. The wave is generated by the heart contraction and its amplitude is a measure of the vessel load and the heart's contraction ability. Thereafter backward propagating waves dominate over forward propagating waves and the intensity change (dI) will consequently be negative. The negative peak expresses the sum of reflection waves from discontinuities in the periphery of the blood vessels [11]. Since the reflection velocity increases in a rigid blood vessel the point of time when the intensity change (dI) becomes negative a good measure of the global elasticity of a blood vessel. The absolute value of the negative wave intensity change (dI) is correlated to AI, Augmentation Index [12], that is an established measure to determine atrial rigidity form changes of the systolic blood pressure. The last characteristic peak is the result of a forward propagating wave having its origin from the closure of the heart valves (aortic valve or pulmonalis valve). In this case there is a decrease in both flow velocity and pressure. The first peak is in early systole, the second in the middle of systole and the last peak is in the end of systole [9]. The interval between the R-wave of the ECG-curve and the first positive peak corresponds to the pre-ejection period. The pre-ejection period decreases when the heart's contraction ability increases. The time interval between the two positive peaks corresponds to the heart's ejection time [7].

To be able to carry out Wave Intensity Analysis of vessels, pressure changes and changes of the blood flow velocity or other parameters correlated thereto must be registered. There exists different method to do this and since the concept first was introduced by Parker et al in 1990 both non-invasive and invasive measurement methods have been developed [4, 8, 10,13-15]. Below is a description of different techniques used to describe how pressure and blood flow are changed. By using these measurement data Wave Intensity Analysis may be performed.

In the beginning the required registration of pressure and flow changes needed to perform Wave Intensity Analysis were invasively performed [4, 8, 10], and e.g. pressure catheters and flow sensors were used. Due to drawbacks of using invasive measurement methods the use on non-invasive methods has gradually been developed in order to measure the changes.

Sugawara et al [13-15] presented a non-invasive technique for Wave Intensity Analysis where instead of measuring pressure changes in the blood vessel, the diameter change of the blood vessel was measured by an ultrasound technique called echo-tracking. The method was calibrated in relation to invasive measurements of systolic and diastolic pressure and a linear relationship between pressure changes and diameter changes was identified for practical implementations [16]. The flow velocity was estimated as the blood velocity measured by the Doppler ultra sound method. The method was patented by ALOKA CO LTD and is further developed by Nike et al [7]. However, this method is limited to measurements of vessels having a diameter larger than a specified value.

The longitudinal direction of a blood vessel is defined by the direction of the blood flow and the radial direction is orthogonal to the longitudinal direction. The above-mentioned methods are based upon the wave propagation that is a result of that the stretching of the vessel wall co-varies with the pressure changes in the vessels, the radial mode, but there are other modes describing wave propagation of the arteries. The longitudinal mode, depending of the viscoelastic characteristics of a moving liquid, describes how waves propagate in vessels in relation to movements of the vessel wall. This mode has not been, or hardly been, possible to observe, due to limitations of image techniques having too low temporal and spatial resolution and the nearly infinitesimal movement of the vessel wall instead was regarded being connected to respiratory movements than to the heart cycle [17]. The fact that the vessel wall really moves in the longitudinal direction during the heart cycle was later proved by Persson et al [18] by using B-mode ultrasound, together with cross correlation. The longitudinal mode has never been used in connection with Wave Intensity Analysis.

Some other suggested methods of measuring the radial and longitudinal movements and velocity of the vessel wall have been presented [20. 21]. By using Doppler technique Sunagawa et al [21] obtained measures of radial and longitudinal velocity and movements, and could at the same time establish that the longitudinal movement of the vessel wall was related to the blood flow close to the wall. There are two problems when measuring the vessel wall movement in two directions, firstly the angle dependency and secondly, the relatively large sampling volume. Large sampling volumes make it difficult of separating movements from different parts of the vessel, and an error of the angle estimation between the longitudinal direction of the vessel and the transmitted ultrasound results in errors of measured velocity values.

By using newly developed high frequency ultrasound techniques and a method based upon block image match, several studies have shown that it is possible to determine longitudinal movement of the vessel wall [19, 20]. Block image matching is a technique that facilitates recognition of a small area in an image in a larger area in a subsequent image.

Methods that measures longitudinal movement of the vessel wall has a large potential in combination with Wave Intensity Analysis to be a tool for diagnosing atherosclerosis at an early stage.

The object of the present application is to achieve a system that improves non invasive methods to characterize blood vessel elasticity. By combining Wave Intensity Analysis by data collecting techniques previously not used in together with Wave Intensity Analysis, a unique combination is achieved that non-invasively solves problems that the presently used techniques cannot solve. According to the new system an intermittent registration of measure values describing the dynamic of the blood vessel wall in longitudinal and radial direction is performed. The system may obtain information of vessel characteristics of peripheral vessels, and the requirement of a specific smallest diameter size to perform the measurements may be discarded. In addition very local measurements of atherosclerosis is possible to perform.

The characterizing features of the present invention are defined by the characterizing portion of claim 1.

## Detailed description of preferred embodiments of the invention

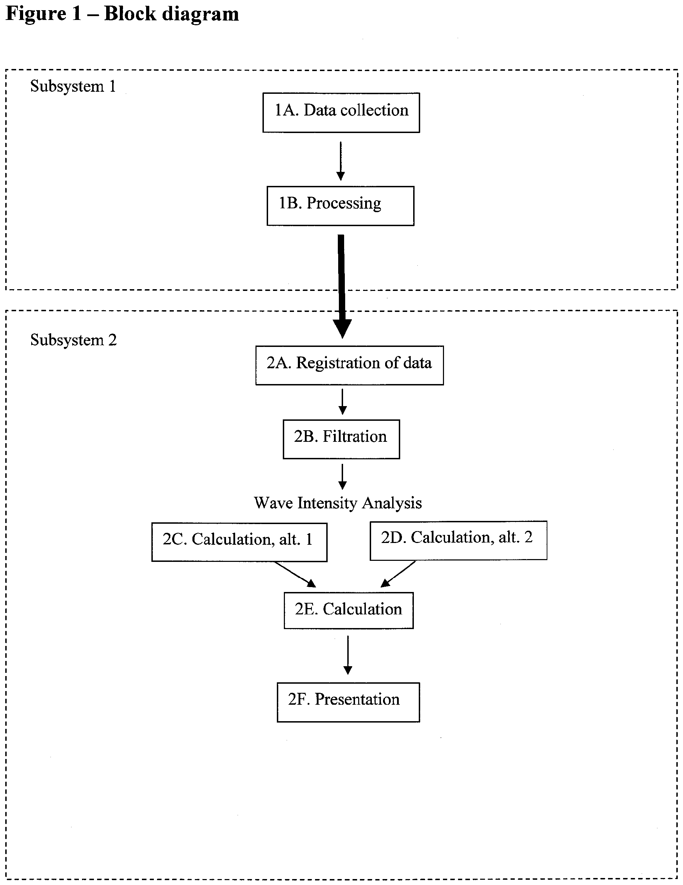

In this part the invention will be described in detail. The invention relates to a non invasive analysis system for global and local blood vessel elasticity. The analysis system comprises two subsystems where subsystem 1 is a data collecting unit and subsystem 2 is an analysis unit. The data collecting unit includes one or many non invasive image generating means, or the data collecting unit facilitates registration of movement parameters quantifying the dynamics of the blood vessel wall in longitudinal and radial direction. There exist different techniques to perform such registrations. One example is the ultrasound technique, which will be described in the following. Subsystem 2, the analysis unit, performs Wave Intensity Analysis based upon movement parameters registered by subsystem 1. The analysis system is shown in figure 1. Subsystem 2 may be separate or may be integrated in subsystem 1.

Subsystem 1 comprises a data collecting unit that registers movement parameters that quantifies the blood vessel wall dynamics in longitudinal and radial direction. The data collecting may e.g. be an ultrasound system, which will be described in detailed below. The movement parameters being registered is the vessel wall velocity (m/s) and the deformation velocity, (strain rate, 1/s).

The ultrasound system comprises an ultrasound probe, an ultrasound device and optionally an analysis program for offline processing of captured ultrasound images. Today there exist two ultrasound techniques to register movement changes of the vessel wall, one reflector-based technique and one technique called tissue-Doppler. Tissue-Doppler is used in all newer ultrasound devices, but reflector-based techniques are relatively new. A reflector-based technique called speckle tracking is e.g. arranged in Vivid7dimentsion from GE Healthcare.

## Reflector-based technique

By the reflector-based technique natural reflectors in the tissue are identified in the gray scale image. By identifying amplitude patterns of the reflected signal the natural reflectors may be trace during a time interval. The technique may measure velocity, movement, deformation and deformation velocity both in axial (parallel to the ultrasound waves) and lateral (orthogonal to the ultrasound waves). These parameters may be extracted for analysis outside the ultrasound system. The advantage of reflector based technique compared to other ultrasound techniques is that the sampling volume is small, which results in a dot formed, local analysis of blood vessel elasticity, and the possibility to measure in small, peripheral vessels. Another advantage is the possibility to follow the same segment of the vessel wall during a time period. Thus, reflector-based techniques then provide for a more true value of the vessel wall velocity and deformation velocity compared to measurement techniques that do not follow natural reflectors in the tissue.

## Tissue-Doppler technique

When an ultrasound wave is reflected by moving tissue or blood the frequency is changed due to the Doppler effect. By the tissue-Doppler ultrasound technique the phase shift of the reflected signal is measured. By using the Doppler equation the tissue velocity then may be analysed and quantified. These velocity values are then extracted to be analysed outside the ultrasound system and the gray scale image is presented in combination with a superimposed colour image where the velocities continuously are colour coded. Tissue-Doppler measure the velocity in the same direction as the direction of the ultrasound which results in that the angle between the ultrasound probe and the blood vessel determines how much the longitudinal and radial velocity component, respectively, contribute to the measured velocity. The angle is estimated and the blood vessel wall velocity is divided into a radial component (orthogonal to the blood-flow) and a longitudinal component (parallel to the blood-flow).

Tissue-Doppler may be measured continuously or intermittently. By continuous Doppler technique echoes from all depth reaches the transducer at the same time, which makes it impossible to separate Doppler signals from different depths. However, a separation would be possible if a pulsed Doppler is used where pulses of ultrasound is emitted and reflected at different points of time from different depths. Pulsed Doppler is considered advantageous in connection with the new analysis system.

## Output data from subsystem 1

The movement parameter(s) should be registered in radial and longitudinal directions, respectively, of a predetermined area of the vessel wall where the vessel elasticity is to be quantified. Commercially available ultrasound systems have functions for offline and manual marking of regions of interest (ROI) in the ultrasound image where registration of movement parameters is performed. An algorithm for automatic placement of ROIs in the vessel wall is a probable further development. Output data from subsystem I is velocity and/or deformation velocity in longitudinal and radial directions in the vessel wall. Output data from subsystem 1 will become input data to subsystem 2.

**Alternative data collecting system**

Subsystem 1 is described in relation to two ultrasound techniques, the reflector-based technique and the tissue-Doppler technique, but any data collecting technique having acceptable temporal and spatial resolution may be used to register values of deformation velocity and velocity in the blood vessel wall. One example of such a data collecting system may be a magneto resonance tomography system. Alternatively may a combination of two or many data collecting systems by used that generates a mean value of the deformation velocity and/or velocity in the longitudinal and radial directions, or to use different data collecting systems for registration of movement parameters in longitudinal and radial direction, respectively, since axial and lateral resolution may differ between different data collecting systems.

**Subsystem 2 - analysing unit**

The object of subsystem 2 is to quantify the blood vessel elasticity. This is performed by Wave Intensity Analysis of the movement parameters registered by subsystem 1, see figure 1. In connection with Wave Intensity Analysis arterial waves are defined by sequential infinitesimal changes of flow and pressure. In subsystem 2, changes of flow and pressure are approximated by movement changes of the blood vessel wall in longitudinal and radial directions, meaning that dU/dt is approximated by the deformation velocity or the velocity of the vessel wall in the longitudinal direction, whereas dP/dt is approximated by the deformation velocity or the velocity in the vessel wall in the radial direction. By using the deformation velocity instead of deformation and velocity instead of movement the value of the intensity change (dI) will be independent of the sampling frequency.

The intensity change (dI) is approximated by the following two expressions:

$$dI = \text{deformation velocity (strain rate) (radial)} * \text{deformation velocity (longitudinal)} * \text{constant}$$

$$dI = \text{velocity (radial)} * \text{velocity (longitudinal)} * \text{constant}$$

The final result of Wave Intensity Analysis includes the graph of the intensity change (dI) and time constants and amplitude values. An estimation of the global and local elasticity of blood vessels is obtained by analysing the graph, but also a measure of the heart's function, e.g. the heart's ejection time. A measure of the global blood vessel elasticity is primarily obtained by analysing time constant, whereas amplitude values are used to obtain information of local elasticity of the blood vessels. In dependence of the design of the data collecting systems in subsystem 1 the intensity change (dI) may be presented in different ways, either as a graph based upon deformation velocity or velocity, or by simultaneous presentation of graphs of intensity change (dI) based upon deformation velocity and velocity.

**Alternative presentation techniques**

A co-variation between deformation velocity and velocity in the blood vessel wall exists but since the deformation velocity relates to the spatial derivate and the velocity relates to the time derivate, the information content is non-redundant. By evaluating the intensity change (dI) by using a combination of deformation velocity and velocity a more accurate indicator of blood vessel elasticity may be obtained. The intensity change (dI) should then be approximated by the following expression:

$$dI(sr) = \text{deformation velocity (radial)} * \text{deformation velocity (longitudinal)} * \text{constant}$$

$$dI(v) = velocity\ (radial) * velocity\ (longitudinal) * constant$$

$$\Rightarrow \quad dI = a * dI(sr) + b * dI(v)$$

where a and b are constants

The input data to subsystem 2 varies depending upon the design of subsystem 1. If subsystem 1 comprises a plurality of data collecting systems resulting in values of movement parameters in longitudinal and radial directions, more curves of intensity changes may be presented. As mentioned above, the intensity change (dI), based upon deformation velocity and velocity, respectively, may be presented simultaneously. The data collecting system may have different axial and lateral resolution resulting in that the longitudinal and radial direction of movement parameters are more accurately registered by using different data collecting systems. A graph of intensity change (dI) may then be calculated by using measurement values from different data collecting systems.

Thus, in the graphical user interface in subsystem 2 the user may choose how many, and which, variants of intensity changes (dI) that should be done based upon the design of subsystem 1. Common for all presentation techniques is that they are based upon values of deformation velocity and velocity measured in the longitudinal and radial directions of the blood vessel.

**Short description of the figure**

Figure 1 is a block diagram describing the flow in the analysis system, having a clear division between subsystem 1 and subsystem 2. The object of the analysis system is to detect global and local vessel elasticity.

**Detailed description of the figure**

**Subsystem 1**

Subsystem 1 is a data collecting unit comprising one or many non-invasive image generating systems, or data collecting system that facilitates registration of movement parameters that quantifies blood vessel wall dynamics in longitudinal and radial directions.

1A. Data collection depending upon chosen data collecting unit.
1B. Processing of collected data to generate movement parameter value as output data. Output data from subsystem 1 will become input data in subsystem 2.

**Subsystem 2**

Subsystem 2 is an analysis unit that performs Wave Intensity analysis based upon movement parameters registered by subsystem 1. Subsystem 2 may be separate, or may be an integral part of subsystem 1.

2A. Receiving movement parameter data from subsystem 1.
2B. Filtration.
2C. Calculation of intensity change (dI) based upon one or many movement parameters registered by one or many data collecting systems. 2C is not necessary if 2D is performed. It depends upon the structure of subsystem 1.
2D. Calculation of intensity change (dI) based upon combination or one or many movement parameters registered by one or many data collecting systems. 2D is not necessary if 2C is performed. It depends upon the structure of subsystem 1.
2E. Calculation of time constants and amplitude values of the intensity change that quantifies global and local blood vessel elasticity.
2F. Presentation of the final result, i.e. one or many intensity change graphs with related time constants and amplitude values.

*References*

1. Boutouyrie, P., et al., Aortic Stiffness Is an Independent Predictor of Primary Coronary Events in Hypertensive Patients: A Longitudinal Study. Hypertension, 2002. 39: p. 10-15.

2. Lehmann, E.D., Pulse wave velocity as a marker of vascular disease. Lancet, 1996. 348: p. 744.

3. Rabben, S.I., et al., An ultrasound-based method for determining pulse wave velocity in superficial arteries. Journal of biomechanical engineering, 2004. 37(10): p. 1615-1622.

4. Parker, K. and C.J.H. Jones, Forward and Backward Running Waves in the Arteries: Analysis Using the Method of Characteristics. Journal of Biomechanical Engineering, 1990. 112(3): p. 322-326.

5. Milnor, W.R., Hemodynamics. 1982, Baltimore: Williams & Wilkins.

6. Nichols, W.W. and M.F. O'Rourke, McDonald's Blood flow in arteries: theoretical, experimental and clinical principles 5ed. 2005, London: Hodder Arnold.

7. Niki, K., et al., A new measurement system for wave intensity: evaluation of carotid arterial wave intensity and reproducibility. Heart Vessels, 2002. 17: p. 12-21.

8. Khir, A.W. and K. Parker, Wave intensity in the ascending aorta: effects of arterial occlusion. Journal of Biomechanics, 2005. 38(4): p. 647-655.

9. Khir, A.W., et al., Determination of wave speed and wave separation in the arteries. Journal of Biomechanics, 2001. 34: p. 1145-1155.

10. Khir, A.W. and K. Parker, Measurements of wave speed and reflected waves in elastic tubes and bifurcations. Journal of Biomechanics, 2002. 35: p. 775-783.

11. Parker, K.H., et al., What stops the flow of blood from the heart? Heart and Vessels, 1988. 4: p. 241-245.

12. Koh, T.W., et al., Analysis of wave reflections in the arterial system using wave intensity: a novel method for predicting timing och amplitude of reflected waves. Heart and Vessels, 1998. 13: p. 103-113.

13. Harada, A., et al., Development of a Non-invasive Real-time Measurement System of Wave Intensity. 2000: p. 1517-1520.

14. Ohte, N., et al., Clinical usefulness of carotid arterial wave intensity in assessing left ventricular systolic and early diastolic performance. Heart Vessels, 2003. 18: p. 107-111.

15. Sugawara, M. and C.J. Jones, Wavefronts in the aorta implications for the mechanisms of the left ventricular ejection and aortic valve closure. Cardiovascular Research 1993. 27: p. 1902-1905.

16. Sugawara, M., et al., Relationship between the pressure and diameter of the carotid artery in humans. Heart Vessels, 2000. 15: p. 49-51.

17. Patel, D.J., A.J. Mallos, and D.L. Fry, Aortic mechanics in the living dog Journal of Applied Physiology, 1961.16: p. 293-299.

18. Persson, M., et al., Non-invasive measurements of arterial longitudinal movement. IEEE Ultrasonics Symposium, 2002: p. 1739-1742.

19. Cinthio, M., et al., Evaluation of an ultrasonic echo-tracking method for measurements of arterial wall movements in two dimensions. IEEE Ultrasonics, ferroelectrics and frequency control society, 2005. 58(8): p. 1300-1311

20. Golemati, S., et al., Carotid artery wall motion estimated from B-mode ultrasound using region tracking and block matching. Ultrasound in Medicine and Biology 2003. 29(3): p. 387-399.

21. Sunagawa, K., H. Kanai, and M. Tanaka, Simulaneous measurement of blood flow and arterial wall vibrations in radial and axial directions. IEEE Ultrasonics Symposium, 2000. 2: p. 1541-1544.

**Claims**

1. Non-invasive analysis system for global and local detection of blood vessel elasticity comprising an analysis unit and a data collecting unit including one or many data collecting systems, wherein said data collecting unit is adapted to register one or many movement parameters, including vessel wall velocity and/or deformation velocity, **characterised in that** said movement parameters quantify the dynamics of the blood vessel wall in both radial and longitudinal directions, of a predetermined area of the vessel wall and that said analysis unit is adapted to perform Wave Intensity Analysis of the registered movement parameters, wherein said analysis unit is adapted to calculate the intensity change of one or many movement parameters, or combinations of one or many movement parameters, and that said analysis unit further is adapted to calculate time constants and amplitude values of the intensity change, that quantifies global and local blood vessel elasticity.

2. System according to claim 1, **characterized in that** said analysis unit is separate or integrated into the data collecting unit.

3. System according to claim 1 or 2, **characterized in that** said movement parameter is the deformation velocity (1/s) of the vessel wall and that the data collecting system is an ultrasound system using the reflector-based technique.

4. System according to claim 1 or 2, **characterized in that** said movement parameter is the vessel wall velocity (m/s) and that the data collecting system is an ultrasound system using the reflector-based technique.

5. System according to claim 1 or 2, **characterized in that** said movement parameter is a combination of the vessel wall velocity (m/s) and the deformation velocity of the vessel wall and that the data collecting system is an ultrasound system using the reflector-based technique.

6. System according to claim 1 or 2, **characterized in that** said movement parameter is the vessel wall velocity (m/s) and that the data collecting system is an ultrasound system using the tissue-Doppler technique.

7. System according to claim 1 or 2, **characterized in that** said data collecting system is a magneto resonance tomography system.

**Patentansprüche**

1. Nichtinvasives Analysesystem für eine globale und lokale Erfassung der Blutgefäßelastizität, umfassend eine Analyseeinheit und eine Datensammeleinheit mit einem oder mehreren Datensammelsystemen, wobei die Datensammeleinheit eingerichtet ist, einen oder mehrere Bewegungsparameter zu registrieren, umfassend eine Gefäßwandgeschwindigkeit und/oder eine Verformungsgeschwindigkeit,

   **dadurch gekennzeichnet, dass** die Bewegungsparameter die Dynamik der Blutgefäßwand in sowohl der Radial- als auch Longitudinalrichtung, eines bestimmten Gebiets der Gefäßwand, quantifizieren und dass die Analyseeinheit eingerichtet ist, eine Wellenintensitätsanalyse der registrierten Bewegungsparameter durchzuführen, wobei die Analyseeinheit eingerichtet ist, die Intensitätsveränderung von einem oder mehreren Bewegungsparametern zu berechnen, oder Kombinationen von einem oder mehreren Bewegungsparametern, und dass die Analyseeinheit ferner eingerichtet ist, Zeitkonstanten und Amplitudenwerte der Intensitätsveränderung zu berechnen, die eine globale und lokale Blutgefäßelastizität quantifiziert.

2. System gemäß Anspruch 1, **dadurch gekennzeichnet, dass** die Analyseeinheit separat oder in die Datensammeleinheit integriert ist.

3. System gemäß Anspruch 1 oder 2, **dadurch gekennzeichnet, dass** der Bewegungsparameter die Verformungsgeschwindigkeit (1/s) der Gefäßwand ist, und dass das Datensammelsystem ein Ultraschallsystem unter Verwendung einer reflektorbasierten Technik ist.

4. System gemäß Anspruch 1 oder 2, **dadurch gekennzeichnet, dass** der Bewegungsparameter die Gefäßwandgeschwindigkeit (m/s) ist, und dass das Datensammelsystem ein Ultraschallsystem unter Verwendung der reflektorbasierten Technik ist.

5. System gemäß Anspruch 1 oder 2, **dadurch gekennzeichnet, dass** der Bewegungsparameter eine Kombination der Gefäßwandgeschwindigkeit (m/s) und der Verformungsgeschwindigkeit der Gefäßwand ist, und dass das Datensammelsystem ein Ultraschallsystem unter Verwendung der reflektorbasierten Technik ist.

6. System gemäß Anspruch 1 oder 2, **dadurch gekennzeichnet, dass** der Bewegungsparameter die Gefäßwandgeschwindigkeit (m/s) ist, und dass das Datensammelsystem ein Ultraschallsystem unter Verwendung der Gewebedopplertechnik ist.

7. System gemäß Anspruch 1 oder 2, **dadurch gekennzeichnet, dass** das Datensammelsystem ein Magnetresonanztomographie-System ist.

## Revendications

1. Système d'analyse non invasif pour la détection globale et locale de l'élasticité d'un vaisseau sanguin comprenant une unité d'analyse et une unité de collecte de données comportant un ou plusieurs systèmes de collecte de données, dans lequel ladite unité de collecte de données est adaptée pour enregistrer un ou plusieurs paramètres de mouvement, dont la vitesse et/ou la vitesse de déformation d'une paroi de vaisseau, **caractérisé en ce que** lesdits paramètres de mouvement quantifient la dynamique de la paroi de vaisseau sanguin dans des directions à la fois radiale et longitudinale, d'une zone prédéterminée de la paroi de vaisseau, et **en ce que** ladite unité d'analyse est adaptée pour réaliser une analyse d'intensité d'onde des paramètres de mouvement enregistrés, dans lequel ladite unité d'analyse est adaptée pour calculer le changement d'intensité d'un ou de plusieurs paramètres de mouvement, ou des combinaisons d'un ou de plusieurs paramètres de mouvement, et **en ce que** ladite unité d'analyse est en outre adaptée pour calculer des constantes de temps et des valeurs d'amplitude du changement d'intensité, qui quantifie l'élasticité globale et locale du vaisseau sanguin.

2. Système selon la revendication 1, **caractérisé en ce que** ladite unité d'analyse est séparée ou intégrée dans l'unité de collecte de données.

3. Système selon la revendication 1 ou 2, **caractérisé en ce que** ledit paramètre de mouvement est la vitesse de déformation (1/s) de la paroi de vaisseau et **en ce que** le système de collecte de données est un système à ultrasons utilisant la technique basée sur un réflecteur.

4. Système selon la revendication 1 ou 2, **caractérisé en ce que** ledit paramètre de mouvement est la vitesse de paroi de vaisseau (m/s) et **en ce que** le système de collecte de données est un système à ultrasons utilisant la technique basée sur un réflecteur.

5. Système selon la revendication 1 ou 2, **caractérisé en ce que** ledit paramètre de mouvement est une combinaison de la vitesse de paroi de vaisseau (m/s) et de la vitesse de déformation de la paroi de vaisseau et **en ce que** le système de collecte de données est un système à ultrasons utilisant la technique basée sur un réflecteur.

6. Système selon la revendication 1 ou 2, **caractérisé en ce que** ledit paramètre de mouvement est la vitesse de paroi de vaisseau (m/s) et **en ce que** le système de collecte de données est un système à ultrasons utilisant la technique Doppler tissulaire.

7. Système selon la revendication 1 ou 2, **caractérisé en ce que** ledit système de collecte de données est un système de tomographie par résonance magnétique.

# Figure 1 – Block diagram

Subsystem 1

1A. Data collection

1B. Processing

Subsystem 2

2A. Registration of data

2B. Filtration

Wave Intensity Analysis

2C. Calculation, alt. 1

2D. Calculation, alt. 2

2E. Calculation

2F. Presentation

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **BOUTOUYRIE, P. et al.** Aortic Stiffness Is an Independent Predictor of Primary Coronary Events in Hypertensive Patients: A Longitudinal Study. *Hypertension,* 2002, vol. 39, 10-15
- **LEHMANN, E.D.** Pulse wave velocity as a marker of vascular disease. *Lancet,* 1996, vol. 348, 744
- **RABBEN, S.I. et al.** An ultrasound-based method for determining pulse wave velocity in superficial arteries. *Journal of biomechanical engineering,* 2004, vol. 37 (10), 1615-1622
- **PARKER, K. ; C.J.H. JONES.** Forward and Backward Running Waves in the Arteries: Analysis Using the Method of Characteristics. *Journal of Biomechanical Engineering,* 1990, vol. 112 (3), 322-326
- **MILNOR, W.R.** Hemodynamics. Baltimore: Williams & Wilkins, 1982
- **NICHOLS, W.W. ; M.F. O'ROURKE.** McDonald's Blood flow in arteries: theoretical, experimental and clinical principles. London: Hodder Arnold, 2005
- **NIKI, K. et al.** A new measurement system for wave intensity: evaluation of carotid arterial wave intensity and reproducibility. *Heart Vessels,* 2002, vol. 17, 12-21
- **KHIR, A.W. ; K. PARKER.** Wave intensity in the ascending aorta: effects of arterial occlusion. *Journal of Biomechanics,* 2005, vol. 38 (4), 647-655
- **KHIR, A.W. et al.** Determination of wave speed and wave separation in the arteries. *Journal of Biomechanics,* 2001, vol. 34, 1145-1155
- **KHIR, A.W. ; K. PARKER.** Measurements of wave speed and reflected waves in elastic tubes and bifurcations. *Journal of Biomechanics,* 2002, vol. 35, 775-783
- **PARKER, K.H. et al.** What stops the flow of blood from the heart?. *Heart and Vessels,* 1988, vol. 4, 241-245
- **KOH, T.W. et al.** Analysis of wave reflections in the arterial system using wave intensity: a novel method for predicting timing och amplitude of reflected waves. *Heart and Vessels,* 1998, vol. 13, 103-113
- **HARADA, A. et al.** *Development of a Non-invasive Real-time Measurement System of Wave Intensity,* 2000, 1517-1520
- **OHTE, N. et al.** Clinical usefulness of carotid arterial wave intensity in assessing left ventricular systolic and early diastolic performance. *Heart Vessels,* 2003, vol. 18, 107-111
- **SUGAWARA, M. ; C.J. JONES.** Wavefronts in the aorta implications for the mechanisms of the left ventricular ejection and aortic valve closure. *Cardiovascular Research,* 1993, vol. 27, 1902-1905
- **SUGAWARA, M. et al.** Relationship between the pressure and diameter of the carotid artery in humans. *Heart Vessels,* 2000, vol. 15, 49-51
- **PATEL, D.J. ; A.J. MALLOS ; D.L. FRY.** Aortic mechanics in the living dog. *Journal of Applied Physiology,* 1961, vol. 16, 293-299
- **PERSSON, M. et al.** Non-invasive measurements of arterial longitudinal movement. *IEEE Ultrasonics Symposium,* 2002, 1739-1742
- **CINTHIO, M. et al.** Evaluation of an ultrasonic echo-tracking method for measurements of arterial wall movements in two dimensions. *IEEE Ultrasonics, ferroelectrics and frequency control society,* 2005, vol. 58 (8), 1300-1311
- **GOLEMATI, S. et al.** Carotid artery wall motion estimated from B-mode ultrasound using region tracking and block matching. *Ultrasound in Medicine and Biology,* 2003, vol. 29 (3), 387-399
- **SUNAGAWA, K. ; H. KANAI ; M. TANAKA.** Simulaneous measurement of blood flow and arterial wall vibrations in radial and axial directions. *IEEE Ultrasonics Symposium,* 2000, vol. 2, 1541-1544